# EUROPÄISCHE PATENTSCHRIFT

(11) **EP 0 488 997 B1**
(45) Veröffentlichungstag und Bekanntmachung des Hinweises auf die Patenterteilung: **17.04.1996**
(21) Anmeldenummer: 92103827.9
(22) Anmeldetag: 30.11.1988
(51) Int. Cl.: A61H 33/02

(54) **Düse für Wirbeldüsenwanne, insbesondere Wirbeldüsenwanne mit selbsttätiger Systemvorspülung**
Nozzle for a whirlpool bath, especially a whirlpool bath with automatic pre-rinsing system
Gicleur pour baignoire à tourbillonnement d'eau, notamment pour baignoire comprenant un système de prérinçage automatique

(30) Priorität: 15.12.1987 DE 3742432
(43) Veröffentlichungstag der Anmeldung: 03.06.1992
(62) Teilanmeldung aus: 88119923.6
(73) Patentinhaber: HOESCH Metall + Kunststoffwerk GmbH & Co., D-52304 Düren (DE)
(72) Erfinder: Klotzbach, Manfred, W-5160 (DE)
(74) Vertreter: Langmaack, Jürgen, Dipl.-Ing.

(56) Entgegenhaltungen:
- EP-A- 0 168 823
- EP-A- 0 215 514
- EP-A- 0 233 753
- WO-A-86/01100
- DE-A- 3 544 002
- US-A- 4 408 721

## Beschreibung

Die Erfindung betrifft eine selbsttätig schließende Düse für die Einleitung eines Wasser-Luft-Gemisches in eine Wanne entsprechend dem Oberbegriff des Anspruchs 1.

Die bisher bekannt gewordenen selbsttätig schließenden Düsen sind wegen der erforderlichen Schwenkbarkeit des Düsenkörpers nicht nur kompliziert in ihrem Aufbau, sondern weisen auch nachteilige Gehäuseformen auf, die eine vollständige Entleerung und/oder eine einwandfreie Durchspülung mit einem Reinigungsmittel nicht gestatten. Ferner ist eine definierte Zuführung der Luft über eine in den Düsenkörper hineinragende Luftleitung nicht möglich, so daß die vorbekannten verschließbaren Düsenformen nur im Saugbetrieb Luft zumischen können, da der Anschluß eines Luftverdichters, also eine unabhängig von der Durchströmung der Düse erfolgende Luftzufuhr nicht möglich ist.

Bei Wannen der eingangs bezeichneten Art mit größerem Füllvolumen, bei denen die Wannen- bzw. Beckenfüllungen über einen längeren Zeitraum beibehalten werden und mehrfach benutzt werden, sind im Rohrleitungssystem Einrichtungen zur Konditionierung des Wassers insbesondere Filter, vorgesehen, um eine Keimbildung im Wasser weitgehend zu unterbinden. Auch bei derartigen Becken ist in gleicher Weise beim Wasserwechsel zunächst für eine vollständige Entleerung auch des Rohrleitungssystems zu sorgen, um bei einer Neufüllung eine Kontaminierung des frisch eingelassenen Wassers zu vermeiden.

Aus der DE-C2 34 20 714 ist eine Wirbeldüsenwanne vorbekannt, bei der das Rohrleitungssystem über ein System von Niveaufühlern und steuerbaren Ventilen so ausgerüstet ist, daß bei einer Befüllung zunächst das gesamte Rohrleitungssystem mit Frischwasser durchspült wird, so daß etwa noch vorhandene Altwasserreste in die Ablaufleitung ausgespült werden und somit bei Inbetriebnahme des im Umwälzverfahren arbeitenden Wirbeldüsensystems nicht mehr in die Wannenfüllung gelangen können.

Es hat sich nun gezeigt, daß die Durchspülung mit Frisch-wasser über einen längeren Zeitraum gesehen nicht immer ausreicht, so daß das gesamte wasserführende Rohrleitungssystem in einem gesonderten Reinigungsgang, bei dem mit dem Frischwasser zugleich eine Reinigungs- und/oder Desinfektionslösung in die Wanne eingefüllt wird, von Zeit zu Zeit behandelt werden muß. Dieser zusätzliche Arbeitsgang wird insbesondere bei Wirbeldüsenwannen, die im privaten Bereich eingesetzt sind, entweder vollständig unterlassen oder in so großen Zeitabständen vorgenommen, daß die Gefahr einer Kontaminierung der Wannenfüllung bei Benutzung über Keimherde im Rohrleitungssystem nicht ausgeschlossen werden kann.

Aus EP-A-0 168 823 ist eine Düse der gattungsgemäßen Art bekannt mit einem Düsenkörper, der in einem Kugelkörper axial verschiebbar geführt ist. Der Kugelkörper ist in einem Düsengehäuse schwenkbar gehalten und weist radiale Öffnungen auf, die mit einer Wasserzuleitung in Verbindung stehen. In dem hohl ausgebildeten Kugelkörper ist eine Luftzuleitung fest angeordnet, die bis in die Bohrung des Düsenkörpers hineinragt, so daß durch Verschieben des Düsenkörpers gegen die Mündung der Luftzuleitung die Wasserzufuhr abgesperrt werden kann. An seinem rückwärtigen Ende steht die Mündung der Luftzuleitung mit einer entsprechenden Versorgungsleitung im Düsengehäuse in Verbindung. Der Düsenkörper ist mit einem Gewinde versehen, so daß die axiale Verschiebung des Düsenkörpers und damit das Öffnen und Verschließen der Wasserzufuhr durch ein Verdrehen von Hand erfolgt. Ein selbsttätiger Verschluß der Düse mit Hilfe des Wasserdruckes ist nicht möglich.

Aus EP-A-0 209 646, Fig. 10, ist eine Düse der gattungsgemäßen Art bekannt, bei der in einem Düsengehäuse ein als Kugelkörper ausgebildeter Düsenkörper schwenkbar gelagert ist. Die Mündung einer mit dem Düsengehäuse fest verbundenen Luftzuleitung endet mit Abstand von der Bohrung im Düsenkörper. Der Luftzuleitung ist eine koaxiale Ringkammer zugeordnet, die mit der Wasserzufuhr in Verbindung steht und in der ein Ringkolbenkörper verschiebbar gelagert ist, der durch Federelemente gegen die Mündung der Luftzuleitung gedrückt wird, so daß der Ringkolbenkörper erst bei Beaufschlagung mit Wasserdruck öffnet und die Wasserzufuhr zur Bohrung des Düsenkörpers unter Mitreißen von Luft aus der Mündung der Luftzuleitung frei wird. Der Nachteil dieser Anordnung besteht darin, daß bei dieser Düsenbauform zunächst ein Wasser-Luft-Gemisch erzeugt wird, das dann in die Bohrung des Düsenkörpers eintritt. Nach einem Verschwenken des Kugelkörpers zur Änderung der Richtung des in die Wanne eintretenden Wasser-Luft-Gemischstrahles muß hierbei der Gemischstrahl umgelenkt werden. Da ein derartiger Gemischstrahl instabil ist, sind die Möglichkeiten einer Richtungsänderung sehr begrenzt. Bei zu starker Richtungsänderung ergibt sich ein Rückstau innerhalb des Düsengehäuses, durch den Wasser in die Luftleitung eingepreßt wird.

Zur Behebung dieser Probleme ist erfindungsgemäß vorgesehen, daß der Haltering als Kolben ausgebildet und axial verschiebbar im Tragring gehalten und auf seiner der Vorkammer abgekehrten Seite über Druckfederelemente abgestützt ist und daß die Außenseite der Mündung der Luftzuleitung als Kugelfläche ausgebildet ist, gegen die der Düsenkörper mit dem Dichtelement seiner Bohrung durch die Federelemente anpreßbar ist. Eine derart ausgebildete Düse mit in den Düsenkörper hineinragender starrer Luftzuleitung ist unabhängig von der Schwenkstellung des kugeligen Düsenkörpers bei druckloser Vorkammer verschlossen. Erst wenn die Druckbeaufschlagung in der Vorkammer durch die Pumpe eine Kolbenkraft bewirkt, die über der Schließkraft der Druckfederelemente liegt, hebt die Dichtung von der Kugelfläche der Mündung der Luftzuleitung ab und gibt die Verbindung zwischen der Vorkammer und dem Wanneninnenraum frei. Die Schließkraft der Druckfederelemente ist hierbei so ausgelegt, daß sie über der Kolbenkraft liegt, die dem Druck in der Vorkammer beim Spülbetrieb entspricht. Insbesondere bei der Verwendung eines nur gering verformbaren Materials für das ringförmige Dichtelement ist es zweckmäßig, wenn der Mittelpunkt der Kugelfläche der Mündung der Luftzuleitung und der Mittelpunkt des Kugelkörpers bei anliegendem Dichtelement zusammenfallen.

In weiterer Ausgestaltung der Erfindung ist vorgesehen, daß die Druckfederelemente sich mit ihren dem Kolben abgekehrten Enden auf einem Stellring abstützen, der in bezug auf die Mündung der Luftzuleitung axial verstellbar ist. Mit Hilfe eines derartigen Stellringes ist es zum einen möglich, unabhängig von der Förderleistung der Pumpe, den Durchtrittsquerschnitt der Düse für das Wasser zu verändern, so daß auch bei der Verwendung von Pumpen mit nicht regelbarer Förderleistung hier eine Beeinflussung der durch die Düsen austretenden Wassermenge möglich ist. Zum anderen ist es möglich, die Düse von Hand auch vollständig zu schließen, so daß der Kugelkörper mit seinem ringförmigen Dichtelement fest gegen die kugelförmige Außenfläche der Mündung der Luftzuleitung gepreßt wird, so daß auch bei einem hohen Druck in der Vorkammer die Düse nicht öffnet. Dies ist insbesondere für Düsen an Wannen mit Systemvorspülung der erfindungsgemäßen Art von Bedeutung. Während im Normalbetrieb, d.h. bei Überschreitung des ersten Druckniveaus sich selbsttätig öffnender Düsen eine Reinigungsmittellösung nur "sanft", d.h. also mit geringer Fließgeschwindigkeit durch das Rohrleitungssystem gefördert werden kann, erlaubt es der über den Stellring mögliche feste, druckunabhängige Verschluß der Düse, das Rohrleitungssystem auch mit der vollen Pumpenleistung zu durchspülen, so daß anstelle oder zusätzlich zu den chemischen Einwirkungen des Reinigungsmittels auch eine mechanische Reinigungswirkung über hohe Strömungsgeschwindigkeiten erzielt werden kann. In zweckmäßiger Ausgestaltung ist daher vorgesehen, daß der Stellring vom Wanneninnenraum her zugänglich ist und wenigstens ein Griffelement aufweist.

In vorteilhafter Ausgestaltung der Erfindung ist ferner vorgesehen, daß der Stellring und der Haltering drehfest, jedoch axial relativ zueinander verschiebbar miteinander verbunden sind. Diese Anordnung hat den Vorteil, daß bei einer Betätigung des Stellringes keine Relativbewegung im Umfangsrichtung erfolgt und somit die Druckfederelemente bei einem Verstellen nicht in Querrichtung beansprucht werden und somit ein Verklemmen oder Verkanten ausgeschlossen ist.

In zweckmäßiger Ausgestaltung der Erfindung ist ferner vorgesehen, daß der die Federelemente umschließende Raum mit einer Entlüftungsöffnung versehen ist. Dies hat den Vorteil, daß die selbsttätige Schließ- und Öffnungsfunktion der Düse auch dann aufrechterhalten bleibt, wenn nach längerer Betriebszeit die Kolbendichtung durchlässig werden und sich zwischen der Vorkammer und dem die Federelemente umschließenden Raum ein Druckausgleich aufbauen würde und dementsprechend auch bei Betriebsdruck die Federelemente den Kolben in Schließstellung halten würden. Über die Entlüftungsöffnung kann somit nicht nur das Luftvolumen in diesem Raum sondern auch eingedrungene Flüssigkeit entweichen, so daß immer eine eindeutige Druckdifferenz zwischen der Vorkammer und dem die Federelemente umschließenden Raum gegeben ist.

In weiterer vorteilhafter Ausgestaltung der Erfindung ist vorgesehen, daß der Kolben an seiner der Vorkammer zugekehrten Kante mit einem Abstreifer versehen ist, der in Schließstellung in die Vorkammer hineinragt. Durch einen derartigen Abstreifer, der auch Teil der Kolbendichtung sein kann, ist gewährleistet, daß beim Drucklossetzen der Anlage sich auf der durch den Tragring gebildeten, zugehörigen Zylinderfläche bildende Kalkablagerungen mechanisch entfernt werden und somit die Leichtgängigkeit des Kolbens in dem den Zylinder bildenden Tragring auch nach längerer Betriebszeit gewährleistet ist.

An Düsen für den Einsatz an Wannen mit der erfindungsgemäßen Systemvorspülung ist in weiterer Ausgestaltung der Erfindung vorgesehen, daß die Vorkammer auf ihrer obenliegenden Seite mit einem Anschlußstutzen für eine Überströmleitung versehen ist. Dies erlaubt es, beim Einfüllen der Reinigungslösung mit Hilfe der Pumpe die Vorkammer vollständig zu entlüften.

In weiterer vorteilhafter Ausgestaltung der Erfindung ist vorgesehen, daß die Vorkammer auf ihrer untenliegenden Seite mit einer Zulauföffnung versehen ist, die wenigstens zwei Abzweigungen aufweist, die vorzugsweise unter einem rechten Winkel zueinander stehen. Diese Anordnung hat für kleinere Wannen, bei denen je Wannenseite nur zwei Düsen vorgesehen sind, den Vorteil, daß die Düsen in gleicher Höhenlage angeordnet werden können, daß andererseits die die beiden Düsen miteinander verbindende Druckleitung sowie die von der Pumpe bis zu der in Strömungsrichtung gesehen ersten Düse geführten Druckleitung praktisch ohne Schräglage verlegt werden kann, so daß beim Entleeren des Systems die Flüssigkeit aus der Vorkammer zur Pumpe zurücklaufen kann. Hierdurch vereinfacht sich die Montage erheblich, da die Anordnung von zusätzlichen Fittings weitgehend entfällt.

In besonders zweckmäßiger Ausgestaltung der erfindungsgemäßen Düse ist ferner vorgesehen, daß die Luftzuleitung unter einem Winkel von oben nach unten verlaufend durch die Vorkammer hindurch in die Bohrung des Düsenkörpers hineinragt. Diese Anordnung hat den Vorteil, daß auch der im Düsenbereich befindliche Teil der Luftzuleitung bei einer Entleerung des Systems vollständig entleert werden kann.

Die Erfindung wird anhand schematischer Zeichnungen an Ausführungsbeispielen näher erläutert. Es zeigen
- Fig. 1: eine Whirlpoolwanne mit Rohrleitungssystem,
- Fig. 2: einen vertikalen Schnitt durch eine Düse,
- Fig. 3: einen Schnitt durch die Vorkammer der Düse gem. Fig. 2 entlang der Linie III-III in Fig. 2,
- Fig. 4: einen Vertikalschnitt durch eine Ablaufarmatur.

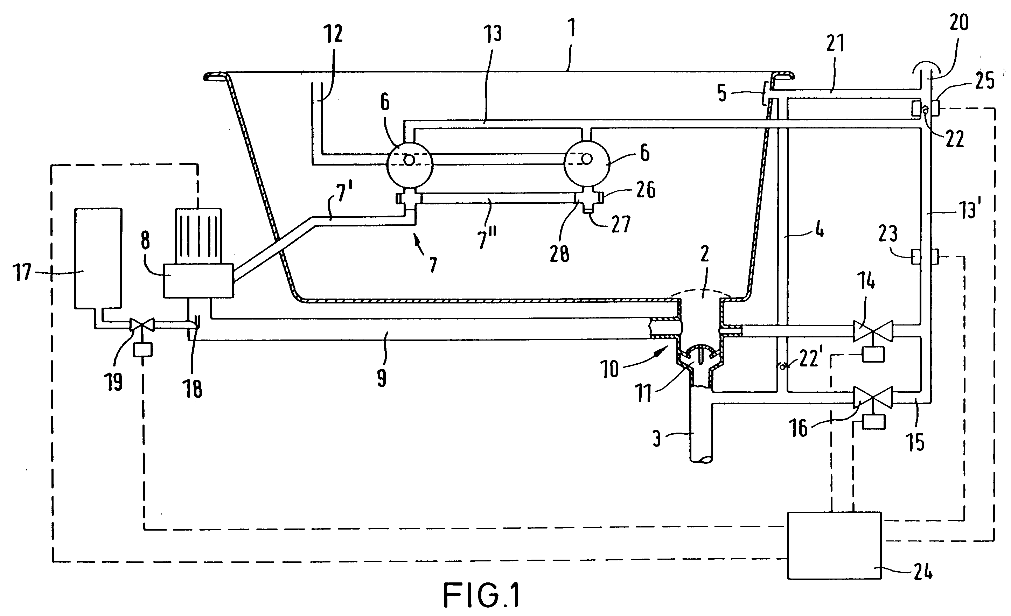

Die in Fig. 1 dargestellte Wirbeldüsenwanne 1 in Form einer etwas größeren Badewanne ist in üblicher Weise mit einer Ablauföffnung 2 im Bodenbereich versehen, die mit einem Ablaufrohr 3 in Verbindung steht. Mit dem Ablaufrohr 3 ist ferner über eine Rohrleitung 4 in üblicher Weise der Wannenüberlauf 5 verbunden.

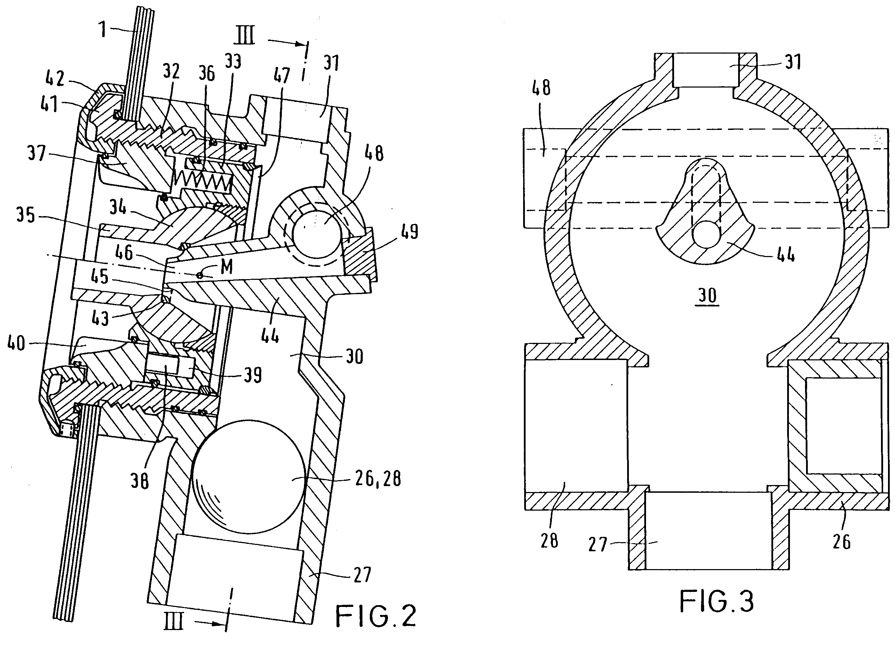

Die Wanne 1 weist nun auf ihren beiden gegenüberliegenden Längsseiten je zwei Düsen 6 auf, durch die ein Wasser-Luft-Gemisch bei gefüllter Badewanne in den Wanneninnenraum strahlförmig eingeleitet werden kann. Die Düsen 6, die anhand von Fig. 2 noch näher erläutert werden, weisen schwenkbare Düsenkörper auf, so daß die Strahlrichtung innerhalb vorgegebener Grenzen frei einstellbar ist.

Die Düsen 6 sind über eine Druckleitung 7 mit der Druckseite einer Pumpe 8 verbunden, die ihrerseits über eine Saugleitung 9 über eine Ablaufarmatur 10 mit der Ablauföffnung 2 in Verbindung steht, die zugleich als Ansaugöffnung für den Pumpenkreislauf dient. Die Ablaufarmatur ist unterhalb der Einmündung der Saugleitung 9 mit einem von außen betätigbaren Ventilkörper 11 gegenüber dem Ablaufrohr 3 verschließbar. Die Pumpe 8 ist hierbei liegend angeordnet, d.h. mit nach unten weisender Ansaugöffnung, so daß bei Stillstand der Pumpe und geöffnetem Ventilkörper 11 die Druckleitung 7 die Pumpe 8 und die Saugleitung 9 vollständig leerlaufen können.

Die Düsen 6 stehen ferner mit einer Luftzuleitung 12 in Verbindung, durch die entweder über die Sogwirkung des in den Wannenraum strömenden Wassers oder aber über einen angeschlossenen Verdichter Luft zusammen mit dem Wasser in Form eines strahlartigen Wasser-Luft-Gemisches in die Wanne eingeleitet werden kann. Der Luftverdichter bzw. die Luftansaugöffnung bei Saugbetrieb, hier nicht näher dargestellt, sind einstellbar, so daß die eingeleitete Luftmenge regulierbar ist.

Die Druckleitung 7 mündet in eine auf der Wannenaußenseite angeordnete Vorkammer der Düsen 6 ein, und zwar von unten. Die Vorkammer jeder Düse 6 ist auf ihrer Oberseite an eine Überströmleitung 13 angeschlossen. Dies wird anhand der Fig. 2 und 3 noch näher erläutert werden.

Die Überströmleitung 13 mündet nun mit ihrem anderen Ende in die Ablaufarmatur 10 oberhalb des Ventilkörpers 11 ein und ist gegenüber dieser über ein Absperrventil 14 absperrbar.

Von der Überströmleitung 13 zweigt eine Zweigleitung 15 ab, die unterhalb des Ventilkörpers 11 in die Ablaufarmatur 10 bzw. das Ablaufrohr 13 einmündet. Auch die Zweigleitung 15 ist über ein Absperrventil 16 gegenüber der Ablaufarmatur bzw. dem Ablaufrohr absperrbar.

Der Wanne ist ferner ein Vorratsbehälter 17 für ein Reinigungs- und/oder Desinfektionsmittel zugeordnet, der über eine Zuleitung 18 vorzugsweise in die Saugleitung 9 einmündet und der über ein Ventil 19 absperrbar ist.

Die Überströmleitung 13 ist ferner mit einem vorzugsweise bis in die Höhe des Wannenrandes geführten Entlüftungsstutzen 20 versehen, der eine Entlüftungsöffnung für die Überströmleitung 13 bildet. Vom Entlüftungsstutzen 20 zweigt eine Querleitung 21 ab, die in die Überlaufleitung 4 einmündet. Durch ein selbsttätiges Ventil, beispielsweise ein Schwimmerventil 22, unterhalb der Querleitung 21 ist sichergestellt, daß nur Leckmengen aus der Überströmleitung 13 in die Überlaufleitung 4 übertreten können.

Der vetikale Teil 13' der Überströmleitung 13 ist mit einem Niveaufühler 23 verbunden, dessen Steuersignal auf eine Steuereinheit 24 aufgeschaltet ist. Dieser Niveaufühler befindet sich gegenüber dem Wannenboden in Höhe einer vorgegebenen Mindestfüllhöhe von beispielsweise 10 cm. Im Entlüftungssutzen 20 ist unterhalb des Ventils 22 ein weiterer Niveaufühler 25 vorgesehen, der ebenfalls auf die Steuereinheit 24 aufgeschaltet ist.

Die Niveaufühler 23 und 25 sind über die Steuereinheite 24 mit dem Antriebsmotor der Pumpe 8 so verschaltet, daß die Pumpe 8 erst eingeschaltet werden kann, wenn die durch den Niveaufühler 23 vorgegebene Mindestfüllhöhe erreicht wird und das auch nur mit einer geringen Förderleistung. Erst bei Erreichen der durch den Niveaufühler 25 vorgegebenen Betriebsfüllhöhe läßt sich die Pumpe mit voller Förderleistung einschalten.

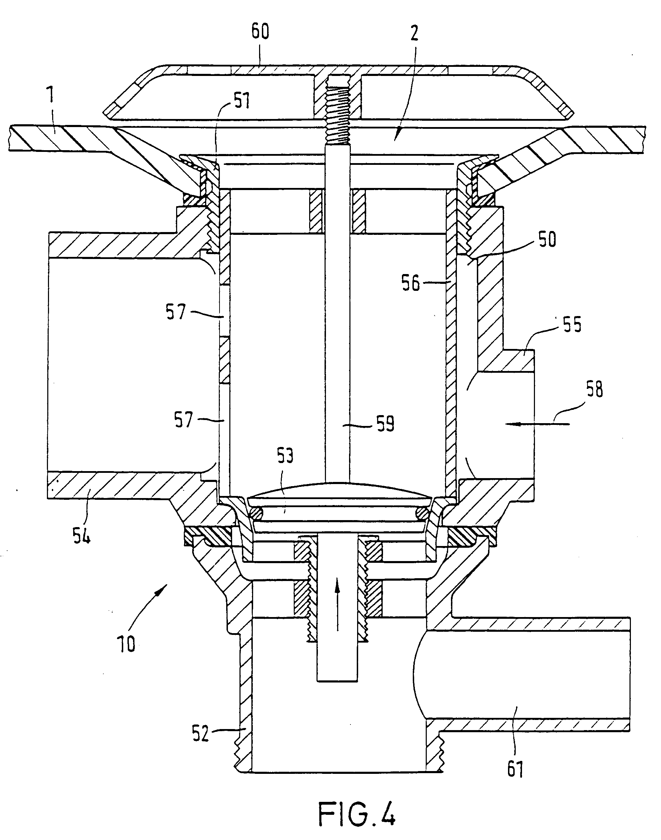

Die Düsen 6 sind verschließbar ausgebildet, was nachstehend anhand von Fig. 2 noch näher erläutert werden wird, so daß bei verschlossenen Düsen 6 und geöffnetem Absperrventil 14 und geschlossenem Absperrventil 16 in der Zweigleitung 15 bei Erreichen der durch den Niveaufühler 23 vorgegebenen Mindestfüllhöhe die Pumpe 8 Flüssigkeit im Kreislauf über die Druckleitung 7, die Überströmleitung 13, 13' und die Saugleitung 9 pumpen kann. Die Ablaufarmatur 10 ist hierbei so ausgebildet, daß keine oder nur geringe Flüssigkeitsmengen aus dem Wanneninnern in die Saugleitung 9 gelangen, was nachstehend anhand von Fig. 4 noch näher erläutert werden wird. Beim Öffnen des Absperrventils 19 kann nun ein Reinigungs- und/oder Desinfektionsmittel zudosiert werden, so daß eine Reinigungsmittellösung im Kreislauf durch das Rohrleitungssystem geführt werden kann, ohne daß der Wanneninnenraum hiermit in Berührung kommt.

Versieht man die Absperrventile 14 und 16 und ggf. das Absperrventil 19 mit steuerbaren Stellantrieben, so lassen sich diese auf die Steuereinheit 24 aufschalten, so daß sowohl die Ansteuerung der Pumpe als auch die Ansteuerung dieser Ventile über die Steuereinheit 24 nach einem vorgegebenen Schaltprogramm erfolgen kann. Hierbei ist es auch möglich, daß während des Reinigungsbetriebes das Absperrventil 16 geringfügig geöffnet wird, so daß immer ein Teilstrom der Reinigungsmittellösung in das Ablaufrohr 3 abgegeben wird und entsprechende Frischwassermengen aus dem Wanneninnenraum in die Saugleitung 9 gelangen. Nach Abschluß der Reinigungsprozedur wird das Absperrventil 16 vollständig geöffnet und das Absperrventil 14 vollständig geschlossen, so daß unter gleichzeitiger Ansaugung von Frischwasser aus dem Wanneninnenraum das wasserführende Rohrleitungssystem, bestehend aus der Saugleitung 9, der Druckleitung 7 und der Überströmleitung 13, 13', mit Frischwasser vollständig durchspült werden kann. Nach Abschluß dieses Spülvorganges wird das Absperrventil 16 geschlossen.

Da mit Beginn des Reinigungsvorganges die Förderpumpe nur mit geringer Förderleistung arbeitet, ist es zudem möglich, das Rohrleitungssystem druckseitig langsam zu füllen, so daß auch die in der Druckleitung 7 sowie in den Vorkammern der Düsen 6 und der Überströmleitung 13 enthaltenen Luftmengen über den Entlüftungsstutzen 20 entweichen können, bis auch das Überströmsystem vollständig gefüllt ist, hierbei ein Rückflußverhinderer 22' in der Rohrleitung 4 und schließlich das Schwimmerventil 22 schließt.

Während des Reinigungsvorganges kann das Befüllen der Wanne weitergeführt werden, da erst bei Erreichen der durch den Niveaufühler 25 vorgegebenen Betriebsfüllhöhe die Pumpe 8 auf volle Leistung schaltbar ist. Über eine entsprechende Verriegelungsschaltung kann sichergestellt werden, daß erst mit Schließen des Absperrventils 16 nach Abschluß des Spülens mit Frischwasser, der Pumpenbetrieb mit voller Leistung möglich ist. Bei von Hand verschlossenen Düsen 6 müssen diese zunächst geöffnet werden. Bei selbsttätig schließenden Düsen reicht der gegenüber dem Reinigungsbetrieb sehr viel höhere Pumpendruck des Wirbelbetriebes aus, die Düsen zu öffnen.

Die Düsen 6 sind im Zulaufbereich mit drei unter einem rechten Winkel zueinander stehenden Anschlußstutzen 26, 27 und 28 versehen, so daß bei der dargestellten Anordnung der zwischen den beiden Düsen verlaufende Teil 7'' der Druckleitung mit den horizontal ausgerichteten Anschlußstutzen 26 und 28 verbunden ist, während der zwischen der Pumpe 8 und der in Strömungsrichtung gesehen ersten Düse liegende Teil 7' der Druckleitung in den nach unten weisenden Anschlußstutzen 27 einmündet. Hierdurch ist ein einwandfreier Ablauf des Wassers beim Entleeren des Rohrleitungssystems gewährleistet.

In Fig. 2 ist nun eine besondere Ausführungsform für eine selbsttätig schließende Düse 6 dargestellt. Die Düse besteht im wesentlichen aus einer Vorkammer 30 mit ihren untenliegenden, horizontal verlaufenden Anschlußstutzen 26, 28 (vgl.Fig. 3) und dem vertikal nach unten weisenden Anschlußstutzen 27. Auf der Oberseite befindet sich ein Anschlußstutzen 31 für den Anschluß der Überströmleitung 13. Die Vorkammer 30 ist gegen die Außenseite der unter einer Neigung von beispielsweise 10° gegenüber der vertikal verlaufenden Seitenwand der Wanne 1 mittels eines an der Innenseite der Wanne eingeschraubten Tragringes 32 unter Abdichtung befestigt. Im Tragring 32, dessen der Vorkammer 30 zugekehrtes Ende als Zylinder ausgebildet ist, ist nun ein als Kolben ausgebildeter und mit Dichtungen versehener Haltering 33 axial verschiebbar geführt.

Im Haltering 33 ist ein als Kugelkörper ausgebildeter Düsenkörper 34 schwenkbar gehalten, dessen rohrförmige, die Strahlrichtung bestimmende Mündung 35 in den Wanneninnenraum weist.

Auf der der Vorkammer 30 abgekehrten Seite ist der Haltering 33 über mehrere gleichmäßig über den Umfang verteilte Druckfederelemente 36 abgestützt, wobei ein Stellring 37 einen Endanschlag definiert. Der Stellring 37 ist ferner mit wenigstens einem Führungsstift 38 versehen, der in eine axiale Bohrung 39 im Haltering 33 eingreift, so daß der Haltering 33 in axialer Richtung relativ gegenüber dem Stellring 37 verschiebbar ist. Der Stellring 37 ist hierbei im Tragring 32 über ein Gewinde gehalten, so daß Stellring und Haltering gemeinsam gegenüber dem Tragring verdreht und axial verschoben werden können. Aufgrund der federnden Ankoppelung zwischen Haltering 33 und Stellring 37 kann hierdurch sowohl das axiale Spiel zwischen verschiebbarem Haltering 33 und Stellring 37 als auch innerhalb gewisser Grenzen die Federvorspannung eingestellt werden. Der Stellring 37 ist hierbei mit einem von der Wanneninnenseite her zugänglichen Griffelement 40 versehen, beispielsweise in Form einer Ausnehmung oder eines Steges. Eine den Klemmbund 41 des Tragringes 32 überdeckende Zierkappe 42 dient zugleich als Begrenzung für die axiale Verstellung des Stellringes 37 in Richtung auf die Wanneninnenseite.

Der im Haltering 33 gelagerte Kugelkörper 34 ist auf der der Vorkammer 30 zugekehrten Seite seiner Bohrung mit einem ringförmigen Dichtelement 43 versehen. Durch die Vorkammer 30 ist im wesentlichen in axialer Richtung, bei dem dargestellten Ausführungsbeispiel geringfügig von oben nach unten geneigt, eine Luftzuleitung 44 geführt, die bis in den Bereich des Düsenkörpers 34 reicht. Die Außenfläche 45 der bis in den Bereich des Düsenkörpers 34 ragenden Mündung 46 der Luftzuleitung 44 ist als Kugelfläche ausgebildet und wirkt mit dem ringförmigen Dichtelement 43 zusammen. Bei drucklosem Zustand der Vorkammer 30 wird der Düsenkörper 34 über die Druckfederelemente 36 mit seinem Dichtelement 43 gegen die Außenfläche 45 gepreßt. Der Mittelpunkt des Kugelteils des Düsenkörpers 34 und die als Kugelfläche geformte Außenfläche 45 der Mündung 46 fallen hierbei zusammen, so daß der Düsenkörper 34 in seinem Haltering 33 verschwenkt werden kann, ohne daß das Dichtelement 43 von der Fläche 45 der Mündung 46 abhebt. In jeder beliebigen Winkelstellung des Düsenkörpers 34 bleibt somit die Abdichtung zwischen dem Wanneninnenraum und der Vorkammer 30 erhalten.

Entsprechend der der Vorkammer 30 zugekehrten Flächenteile des Halteringes 33 und des Düsenkörpers 34 bewirkt nun jede Druckbeaufschlagung der Vorkammer 30 eine gegen die Kraft der Druckfederelemente gerichtete Kolbenkraft. Die Kolbenkraft kann jedoch die Schließkraft der Druckfederelemente 36 erst ab einer bestimmten, vorgegebenen Druckhöhe überwinden und den Düsenkörper 34 mit seiner Dichtung 43 von der Gegenfläche 48 der Mündung 46 abheben, so daß aus der Vorkammer 30 das Wasser in den Wanneninnenraum strömen kann. Für eine Wanne mit vorspülbarem Leitungssystem ist die Schließkraft der Druckfederelemente 36 so eingestellt, daß bei einer geringen Mindestförderleistung der zugehörigen Pumpe die Düsen sicher verschlossen sind und erst nach Einschalten der sehr viel höheren Förderleistung für den Wirbelbetrieb die Düsen sicher geöffnet sind.

Durch Verdrehen des Stellringes 37 in Richtung auf die Vorkammer 30 ist es jedoch möglich, die druckabhängige Axialbewegung des Halteringes 33 zu unterbinden und den Düsenkörper 34 gegen die Mündung 46 anzupressen, so daß das Rohrleitungssystem auch mit der vollen Förderleistung der Pumpe durchspült werden kann.

Der als Kolben ausgebildete Haltering 33 ist an seinem der Vorkammer 30 zugekehrten Kantenbereich mit einem Abstreifer 47 versehen, der bei druckloser Vorkammer 30 in diese hineinragt, so daß Kalkablagerungen von der durch den Tragring 32 gebildeten Zylinderfläche abgeschoben werden bzw. nicht entstehen können.

Wenn der Düsenkörper 34 dichtend an der Mündung 46 anliegt, ist zwar die Verbindung zwischen dem Wanneninnenraum und der Vorkammer 30 unterbrochen. Gleichwohl kann bei gefüllter Wanne Wasser in die Luftzuleitung 44 einlaufen. Durch die geneigte Anordnung der Luftzuleitung 44 wird diese jedoch beim Entleeren der Wanne zuverlässig entleert. Bei dem dargestellten Ausführungsbeispiel erfolgt die Verbindung mit der Luftleitung 12 durch seitliche Anschlußstutzen 48, so daß zusätzlich noch die Möglichkeit eines axialen Anschlusses verbleibt, beispielsweise für einen zusätzlichen Luftverdichter. Bei dem dargestellten Ausführungsbeispiel ist die axiale Zuleitung jedoch durch einen Stopfen 49 geschlossen.

Wie in Fig. 3 dargestellt, werden nicht benötigte Anschlußstutzen durch einen Stopfen verschlossen.

Wie Fig. 2 zeigt, sind die beweglichen Teile der Düse jederzeit vom Wanneninnraum her zugänglich, ohne daß die Anordnung von der Wanne gelöst werden muß. Hierzu ist es lediglich erforderlich, die Zierkappe 42 zu lösen und dann den Stellring 37 zusammen mit dem als Kolben ausgebildeten Haltering 33 herauszuziehen. Die Dichtungen der gegeneinander bewegbaren Teile lassen sich dann erneuern oder ein neues Kolbenteil einsetzen.

In Fig. 4 ist ein Ausführungsbeispiel für die Ablaufarmatur 10 dargestellt. Diese weist eine Strömungskammer 50 auf, die über einen Klemmring 51 mit der Ablauföffnung 2 im Boden der Wanne 1 fest verbunden ist. Die Strömungskammer 50 weist an ihrem unteren Ende einen Anschlußstutzen 52 auf, mit dem sie an die Ablaufleitung 3 (hier nicht dargestellt) angeschlossen ist. Im unteren Bereich der Strömungskammer 50 ist ein Ventilkörper 53 angeordnet, der aus der dargestellten Schließstellung über einen hier nicht näher dargestellten Öffnungsmechanismus in die Öffnungsstellung angehoben werden kann.

Die Strömungskammer 50 ist oberhalb der Ebene des Vemtilkörpers 53 mit einer Strömungsöffnung 54 mit großem Durchmesser in Form eines Anschlußstutzens versehen, der eine zweite Stömungsöffnung 55, ebenfalls in Form eines Anschlußstutzens, diametral gegenüberliegend angeordnet ist. Die Strömungsöffnung 55 hat einen geringeren Strömungsquerschnitt als die Strömungsöffnung 54. An die Strömungsöffnung 54 wird die Saugleitung 9 der Pumpe 8 und an die Strömungsöffnung 55 wird die Überströmleitung 13 des anhand von Fig. 1 beschriebenen Rohrleitungssystems angeschlossen.

In der Strömungskammer 50 ist ein Rohreinsatz 56 angeordnet, der einen geringeren Durchmesser aufweist, als die Strömungskammer selbst und der auf seiner der mit der Saugleitung 9 verbindbaren Strömungsöffnung 54 zugekehrten Seite mit einer Vielzahl von Öffnungen 57 versehen ist, während er auf seiner der Strömungsöffnung 55 zugekehrten Seite eine vollständig geschlossene Wandung aufweist. Bei entsprechender Bemessung der Querschnitte wird hierdurch vermieden, daß das beim Spülvorgang in Richtung des Pfeiles 58 über die Strömungsöffnung 55 zuströmende Wasser in den Wanneninnenraum eintreten kann.

Der Ventilkörper 53 weist auf seiner Oberseite eine Verlängerung 59 auf, mit der eine mit Löchern versehene Abdeckkappe 60 verbunden ist. Bei geschlossenem Ventilkörper dient diesen Abdeckkappe 60 als Sicherung der Zulauföffnung zur Saugleitung 9. Beim Öffnen des Ventilkörpers wird die Abdeckkappe 60 mit angehoben, so daß ein ausreichender Querschnitt für den Entleerungsvorgang vorhanden ist. Die Löcher 57 im Rohreinsatz 56 sind hierbei so angeordnet, daß auch die mit den Öffnungen 55 und 54 verbundenen Zuleitungen vollständig über die Ablaufarmatur 10 entleerbar sind.

Unterhalb des Ventilkörpers 53 ist eine weitere Strömungsöffnung 61, ebenfalls in Form eines Anschlußstutzens vorgesehen, in die bei einer Anordnung gem. Fig. 1 die Zweigleitung 15 einmündet.

## Patentansprüche

1. Selbsttätig schließende Düse für die Einleitung eines Wasser-Luft-Gemisches in eine Wanne, mit einem Tragring (32), über den eine mit einer Druckleitung verbundene Vorkammer (30) fest mit der Wannenwand (1) verbunden ist, in die von der Wannenaußenseite her die Mündung (46) einer Luftzuleitung (44) hineinragt, wobei mit dem Tragring (32) ein Haltering (33) lösbar verbunden ist, in dem ein als Kugelkörper ausgebildeter Düsenkörper (34) schwenkbar gehalten ist, der eine Bohrung aufweist, die mit der Vorkammer (30) verbunden ist und die an ihrem der Mündung (46) der Luftzuleitung (44) zugekehrten Ende mit einem die Bohrung begrenzenden ringförmigen Dichtelement (34) versehen ist, dadurch gekennzeichnet, daß der Haltering (33) als Kolben ausgebildet und axial verschiebbar im Tragring (32) gehalten und auf seiner der Vorkammer (30) abgekehrten Seite über Druckfederelemente (36) abgestützt ist und daß die Außenseite der Mündung (46) der Luftzuleitung (44) als Kugelfläche (45) ausgebildet ist, gegen die der Düsenkörper (34) mit dem Dichtelement (43) seiner Bohrung durch die Federelemente (36) anpreßbar ist.

2. Düse nach Anspruch 1, dadurch gekennzeichnet, daß der Mittelpunkt (M) der Kugelfläche (45) der Mündung (46) der Luftzuleitung (44) und der Mittelpunkt des Kugelkörpers (34) bei anliegendem Dichtelement (43) zusammenfallen.

3. Düse nach Anspruch 1 oder 2, dadurch gekennzeichnet, daß die Druckfederelemente (36) mit ihren der Vorkammer (30) abgekehrten Enden auf einem Stellring (37) abstützen, der in bezug auf die Mündung (46) der Luftzuleitung (44) axial verstellbar ist.

4. Düse nach einem der Ansprüche 1 bis 3, dadurch gekennzeichnet, daß der Stellring (37) vom Wanneninnenraum her zugänglich ist und wenigstens ein Griffelement (40) aufweist.

5. Düse nach einem der Ansprüche 1 bis 4, dadurch gekennzeichnet, daß der Stellring (37) und der Haltering (33) drehfest jedoch axial relativ zueinander verschiebbar miteinander verbunden sind.

6. Düse nach einem der Ansprüche 1 bis 5, dadurch gekennzeichnet, daß der die Federelemente (36) umschließende Raum mit einer Entlüftungsöffnung versehen ist.

7. Düse nach einem der Ansprüche 1 bis 6, dadurch gekennzeichnet, daß der kolbenförmige Haltering (33) an seiner der Vorkammer (30) zugekehrten Kante mit einem Abstreifer (47) versehen ist, der in Schließstellung in die Vorkammer (30) hineinragt.

8. Düse nach einem der Ansprüche 1 bis 7, dadurch gekennzeichnet, daß die Vorkammer (30) auf ihrer obenliegenden Seite mit einem Anschlußstutzen (31) für eine Überströmleitung versehen ist.

9. Düse nach einem der Ansprüche 1 bis 8, dadurch gekennzeichnet, daß die Vorkammer (30) auf ihrer untenliegenden Seite mit einer Zulauföffnung versehen ist, die wenigstens zwei Abzweigungen (26, 27, 28) aufweist, die vorzugsweise unter einem rechten Winkel zueinander stehen.

10. Düse nach einem der Ansprüche 1 bis 9, dadurch gekennzeichnet, daß die Luftzuleitung (44) unter einem Winkel von oben nach unten verlaufend durch die Vorkammer (30) hindurch in die Bohrung des Düsenkörpers (34) hineinragt.

## Claims

1. An automatically closing nozzle for the introduction of a water/air mixture into a trough, with a bearing ring (32), via which a pre-chamber (30), connected with a pressure line, is firmly connected with the trough wall (1), into which, from the direction of the outer side of the trough, the opening (46) of an air feed line (44) projects, in which a retaining ring (33) is releasably connected with the bearing ring (32), in which retaining ring (33) a nozzle body (34), constructed as a spherical body, is swivellably held, which has a bore which is connected to the pre-chamber (30) and which at its end facing the opening (46) of the air feed line (44) is provided with an annular sealing element (34) delimiting the bore, characterised in that the retaining ring (33) is constructed as a piston and is held so as to be axially displaceable in the bearing ring (32) and on its side facing away from the pre-chamber (30) is supported via compression spring elements (36) and that the outer side of the opening (46) of the air feed line (44) is constructed as a spherical surface (45), against which the nozzle body (34) is able to be pressed with the sealing element (43) of its bore through the spring elements (36).

2. A nozzle according to Claim 1, characterised in that the central point (M) of the spherical surface (45) of the opening (46) of the air feed line (44) and the central point of the spherical body (34) coincide when the sealing element (43) lies adjacent.

3. A nozzle according to Claim 1 or 2, characterised in that the compression spring elements (36) bear with their ends facing away from the pre-chamber (30) on an adjusting ring (37) which is axially adjustable in relation to the opening (46) of the air feed line (44).

4. A nozzle according to one of Claims 1 to 3, characterised in that the adjusting ring (37) is accessible from the interior of the trough and has at least one grip element (40).

5. A nozzle according to one of Claims 1 to 4, characterised in that the adjusting ring (37) and the retaining ring (33) are connected with each other so as to be rotationally secure but displaceable axially relative to each other.

6. A nozzle according to one of Claims 1 to 5,
characterised in that the space surrounding the spring elements (36) is provided with a ventilation opening.

7. A nozzle according to one of Claims 1 to 6, characterised in that the piston-shaped retaining ring (33) is provided on its edge facing the pre-chamber (30) with a stripper (47) which projects in the closed position into the pre-chamber (30).

8. A nozzle according to one of Claims 1 to 7, characterised in that the pre-chamber (30) is provided on its side lying above with a connection piece (31) for an overflow pipe.

9. A nozzle according to one of Claims 1 to 8, characterised in that the prechamber (30) is provided on its side lying underneath with an inlet opening which has at least two branches (26, 27, 28), which are preferably at right-angles to each other.

10. A nozzle according to one of Claims 1 to 9, characterised in that the air feed line (44) projects at an angle running from top to bottom through the pre-chamber (30) into the bore of the nozzle body (34).

## Revendications

1. Buse se fermant automatiquement pour l'introduction d'un mélange air-eau dans une baignoire, comportant une bague-support (32), par laquelle une préchambre (30) reliée à une canalisation de pression est fixée à la paroi de baignoire (1), préchambre dans laquelle l'embouchure (46) d'une canalisation d'amenée d'air (44) fait saillie depuis la paroi extérieure de baignoire, une bague de retenue (33) démontable étant liée à la bague-support (32), bague de retenue dans laquelle un corps de buse (34) agencé sous forme de corps sphérique est maintenu pivotant , corps qui comporte un alésage relié à la préchambre (30) et qui est muni à son extrémité tournée vers l'embouchure (46) de la canalisation d'arrivée d'air (44) d'un élément d'étanchéité annulaire (43) délimitant l'alésage, caractérisée par le fait que la bague de retenue (33) est réalisée en tant que piston et est maintenue coulissante axialement dans la bague-support (32) et est poussée sur son côté éloigné de la préchambre (30) par des éléments élastiques de pression (36), et par le fait que le côté extérieur de l'embouchure (46) de la canalisation d'arrivée d'air (44) est réalisé en tant que surface sphérique (45), contre laquelle le corps de buse (34) peut être appliqué avec l'élément d'étanchéité (43) de son alésage par les éléments élastiques (36).

2. Buse selon la revendication 1, caractérisée par le fait que le centre (M) de la surface sphérique (45) de l'embouchure (46) de la canalisation d'arrivée d'air (44) et le centre du corps sphérique (34) coïncident lorsque l'élément d'étanchéité (43) est appliqué.

3. Buse selon la revendication 1 ou 2, caractérisée par le fait que les éléments élastiques de pression (36) sont en appui à leurs extrémités éloignées de la préchambre (30) contre une bague de réglage (37) , qui est réglable axialement par rapport à l'embouchure (46) de la canalisation d'arrivée d'air (44).

4. Buse selon l'une des revendications 1 à 3, caractérisée par le fait que la bague de réglage (37) est accessible depuis l'espace intérieur de la baignoire et comporte au moins un élément de prise (40).

5. Buse selon l'une des revendications 1 à 4, caractérisée par le fait que la bague de réglage (37) et la bague de retenue (33) sont reliées l'une à l'autre de manière fixe en rotation tout en pouvant coulisser axialement l'une par rapport à l'autre.

6. Buse selon l'une des revendications 1 à 5, caractérisée par le fait que l'espace entourant les éléments élastiques (36) est muni d'un orifice de ventilation.

7. Buse selon l'une des revendications 1 à 6, caractérisée par le fait que la bague de retenue (33) en forme de piston est munie sur son arête tournée vers la préchambre (30) d'un racleur (47) qui fait saillie dans la préchambre (30) en position de fermeture.

8. Buse selon l'une des revendications 1 à 7, caractérisée par le fait que la préchambre (30) est munie sur son côté supérieur d'un raccord (31) pour une canalisation de trop-plein.

9. Buse selon l'une des revendications 1 à 8, caractérisée par le fait que la préchambre (30) est munie sur son côté inférieur d'un orifice d'amenée qui comporte au moins deux branches (26, 27, 28), lesquelles sont avantageusement disposées à angle droit l'une par rapport à l'autre.

10. Buse selon l'une des revendications 1 à 9, caractérisée par le fait que la canalisation d'arrivée d'air (44) fait saillie dans l'alésage du corps de buse (34) en s'étendant à travers la préchambre (30) sous un angle du haut vers le bas.
